(19) Europäisches Patentamt
European Patent Office
Office européen des brevets

(11) **EP 1 724 346 B1**

(12) **EUROPÄISCHE PATENTSCHRIFT**

(45) Veröffentlichungstag und Bekanntmachung des Hinweises auf die Patenterteilung:
**04.06.2008 Patentblatt 2008/23**

(51) Int Cl.:
*C12N 15/09* (2006.01)     *C07K 7/08* (2006.01)
*A61K 38/10* (2006.01)

(21) Anmeldenummer: **06009706.0**

(22) Anmeldetag: **11.05.2006**

(54) **An die Lck-SH3 und Hck-SH3 Domäne bindendes Peptid**

Peptide binding to the Lck-SH3 and to the Hck-SH3 domain

Peptide se fixant au domaine de type Lck-SH3 et Hck-SH3

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **19.05.2005 DE 102005023761**

(43) Veröffentlichungstag der Anmeldung:
**22.11.2006 Patentblatt 2006/47**

(73) Patentinhaber: **Forschungszentrum Jülich GmbH 52425 Jülich (DE)**

(72) Erfinder:
• **Willbold, Dieter**
  **52457 Aldenhoven (DE)**
• **Hoffmann, Silke**
  **61250 Usingen (DE)**
• **Wiesehan, Katja**
  **52428 Jülich (DE)**
• **Tran, Thi Tuyen**
  **48231 Warendorf (DE)**

(56) Entgegenhaltungen:
• SCHWEIMER KRISTIAN ET AL: "Structural investigation of the binding of a herpesviral protein to the SH3 domain of tyrosine kinase Lck" BIOCHEMISTRY, Bd. 41, Nr. 16, 23. April 2002 (2002-04-23), Seiten 5120-5130, XP002398780 ISSN: 0006-2960
• HORITA DAVID A ET AL: "Solution structure of the human Hck SH3 domain and identification of its ligand binding site" JOURNAL OF MOLECULAR BIOLOGY, Bd. 278, Nr. 1, 24. April 1998 (1998-04-24), Seiten 253-265, XP004453992 ISSN: 0022-2836
• GREENWAY ALISON ET AL: "Human immunodeficiency virus type 1 Nef binds directly to Lck and mitogen-activated protein kinase, inhibiting kinase activity" JOURNAL OF VIROLOGY, Bd. 70, Nr. 10, 1996, Seiten 6701-6708, XP002398781 ISSN: 0022-538X
• ALEXANDROPOULOUS KONSTANTINA ET AL: "Proline-rich sequences that bind to Src homology 3 domains with individual specificities" PROCEEDINGS OF THE NATIONAL ACADEMY OF SCIENCES OF THE UNITED STATES OF AMERICA, Bd. 92, Nr. 8, 1995, Seiten 3110-3114, XP002398782 ISSN: 0027-8424
• BRIGGS SCOTT D ET AL: "The Ras GTPase-activating Protein (GAP) Is an SH3 Domain-binding Protein and Substrate for the Src-related Tyrosine Kinase, Hck" JOURNAL OF BIOLOGICAL CHEMISTRY, Bd. 270, Nr. 24, 1995, Seiten 14718-14724, XP002398783 ISSN: 0021-9258
• LEE C-H ET AL: "A single amino acid in the SH3 domain of Hck determines its high affinity and specificity in binding to HIV-1 Nef protein" EMBO JOURNAL, OXFORD UNIVERSITY PRESS, SURREY, GB, Bd. 14, Nr. 20, 1995, Seiten 5006-5015, XP002236097 ISSN: 0261-4189
• TRAN TUYEN ET AL: "Insights into human Lck SH3 domain binding specificity: Different binding modes of artificial and native ligands" BIOCHEMISTRY, Bd. 44, Nr. 45, November 2005 (2005-11), Seiten 15042-15052, XP002430256 ISSN: 0006-2960

**Beschreibung**

Cytoplasmatische Tyrosin-spezifische Proteinkinasen (PTKs) sind intrazelluläre Effektormoleküle, die an verschiedenen Signalübertragungswegen beteiligt sind.

PTKs können dabei über ihre nicht-katalytischen- SH3- und SH2-Domänen mit bestimmten Subtratproteinen assoziieren und diese dann im Sinne einer Signalweitergabe durch eine Tyrosinphosphorylierung aktivieren. Die menschliche T-Zell Tyrosinkinase (Lck) nimmt eine Schlüsselrolle bei der Signalübertragung in T-Zellen ein und ist essentiell für die Entwicklung und Funktion dieser Zellen. Krankheiterreger, wie das menschliche Immunschwächevirus (HIV) oder Herpesvirus saimiri, haben Effektormoleküle entwickelt, die mit Lck wechselwirken, eine geregelte Immunantwort verhindern und so z. B. ihre eigene Vermehrung gewährleisten. Die Bindung der viralen Proteine an die Lck wird dabei durch die Interaktion einer Prolin-reichen Region (PPHII) im Virusprotein mit der SH3-Domäne der Lck vermittelt.

Von einer großen Anzahl von SH3 Domänen wurde die Spezifität mittlerweile mittels Phagendisplay oder anderen Methoden von verschiedenen Arbeitsgruppen untersucht. Beispielhaft kann die Veröffentlichung von Sparks AB, Rider JE, Hoffmann NG, Fowlkes DM, Quillam LA, Kay BK "Distict ligand preferences of Src homology 3 domains from Src, Yes, Abl, Cortactin, p53bp2, PLCgamma, Crk and Grb2" genannt werden. Die Ergebnisse dieser Arbeiten sowie die Ergebnisse sehr aufwendiger, systematischer Arbeiten an ganzen Genomen (z. B. Hefe) haben aber bis zum heutigen Tage zu keinem verlässlichen Erkennungscode für SH3-Domänen geführt, dass heißt, es besteht bisher keine Möglichkeit systematisch zu erkennen, welche Polyprolinhaltige Peptide an die SH3-Domäne der Lck binden. Allerdings sind Peptide aus natürlichen Liganden der Lck SH3 bekannt, die an die Lck-SH3 binden, wie in Poghosyan Z. et al. in Phosphorylationdependent interactions between ADAM15 cytoplasmic domain and Scr family protein-tyrosine kinase J Biol Chem. 2002 Feb 15; 277(7):4999-5007 veröffentlicht.

Es ist die Aufgabe der Erfindung, Liganden zu identifizieren, die eine möglichst affine Bindung an die Lck-SH3 Domäne eingehen und vorzugsweise in der Lage sind, die Bindung z. B. viraler Effektormoleküle an die Lck-SH3 zu unterbinden oder eine schon erfolgte Bindung wieder aufzulösen. Weiterhin soll ein Wirkstoff zur Verfügung gestellt werden, welcher dazu in der Lage ist, die Immunabwehr zu beeinflussen.

Erfindungsgemäß wird die Aufgabe durch die Bereitstellung der in dem Sequenzprotokoll 1 angegebenen Peptid und dessen Verwendung zur Besetzung der SH3-Domäne von Tyrosinkinase (Lck) von Immunzellen sowie die DNA gemäß Sequenzprotokoll 2 gelöst.

Erfindungsgemäß wird die SH3-Domäne von Lck mit dem erfindungsgemäßen Peptid nach dem Sequenzprotokoll 1 mit einer hohen Affinität besetzt.

Das Peptid gemäß Seq. 1 bindet zusätzlich an die Hck-SH3 Domäne, was den Vorteil hat, die Bindung z. B. viraler Effektormoleküle an die Hck-SH3 zu unterbinden oder eine solche Bildung aufzulösen.

Das erfindungsgemäße Peptid bindet besonders bevorzugt an die Lck- SH3-Domänen vom Menschen (Homo sapiens), von der Maus (Mus musculus), der Ratte (Rattus norvegicus), dem Hund (Canis familiaris), dem Nachtaffe (Aotus nancymaae) oder dem Schimpansen (Pan troglodytes) an. Allerdings ist die Anbindung nicht auf diese Spezies beschränkt.

Weiterhin bindet das erfindungsgemäße Peptid beispielhaft, aber nicht beschränkend an die Hck-SH3-Domäne vom Menschen (Homo sapiens), dem Schimpansen (Pan troglodytes), dem Langschwanzmakak (Macaca fascicularis) und dem Hund (canis familiaris) an.

Besonders bevorzugt bindet das erfindungsgemäße Peptid an die Lck- und Hck- SH3 von Spezies an, deren Sequenz mit der menschlichen Sequenz identisch ist.

Die Besetzung der SH3-Domäne der Lck und/oder der Hck mit den erfindungsgemäßen Protein bzw. Peptid gemäß Sequenzprotokoll 1 ermöglicht es, eine Abstoßung von bei Patienten transplantierten Organen zu verhindern oder zumindest die Wahrscheinlichkeit einer Organabstoßung zu reduzieren.

Weiterhin können für den Organismus gefährliche Viren, wie HIV oder Hepatitis C in ihrer Wirkung geschwächt oder sogar vollkommen desaktiviert werden, da deren Effektormoleküle nicht mehr an die SH3-Domäne der Lck oder Hck binden und damit die Immunabwehr behindern bzw. außer Kraft setzen können. Eine Behandlung von mit den entsprechenden Viren assoziierten Krankheiten ist daher möglich. Beispielsweise kann bei einer HIV-1 Infektion AIDS bzw. bei einer Hepatitis C Infektion eine virusbedingte Hepatitis behandelt werden.

Die erfindungsgemäßen Peptide können als Bestandteil von in-vitro/in-vivo Aktivitätstests für Kinasen verwendet werden, die die Aufklärung von Signalwegen in der biomedizinischen Grundlagenforschung ermöglichen.

Das Immunsystem kann durch die Besetzung der Lck und/oder der Hck SH3 Domäne durch das erfindungsgemäße Peptid gemäß Seq.1 beeinflusst werden.

Wird eine Schwächung des Immunsystems hervorgerufen, kann eine Abstoßung von bei Patienten transplantierten Organen verhindert oder zumindest die Wahrscheinlichkeit einer Abstoßung nach deren Transplantation reduziert werden. Ebenfalls können Autoimmunkrankheiten wie Multiple Sklerose, systemischer Lupus erythematodes, insulinabhängiger Diabetes mellitus, rheumatische Arthritis, chronische Thyreoiditis, Spondylitis ankylosans, akute anteriore Uveitis, Goodpasteure Syndrom, Basedow Krankheit, Myasthenia gravis und Pemphigus vulgaris therapiert werden. Wir eine Stärkung des Immunsystems durch das erfindungsgemäße Peptid gemäß Seq. 1 hervorgerufen, ist eine begleitende Therapie mit aus dem Peptid abgeleiteten Wirkstoffen bei sämtlichen Krankheiten möglich, deren Verlauf durch eine gestärkte Immunabwehr abgeschwächt werden kann (z. B. Krebs, bakterielle und virale Infektionen im Allgemeinen).

Die Erfindung umfasst auch Arzneimittel, welche das erfindungsgemäße Peptid gemäß Sequenzprotokoll 1 umfassen.

Mit den Arzneimitteln können die Krankheiten Aids oder Erkrankungen, die durch das Virus Hepatitis C bedingt sind und die oben genannten Autoimmunkrankheiten behandelt werden.

Eine sehr effektive Nef-Verdrängung von der Hck SH3 Domäne erfolgte durch PD1 (Seq. 1).

Das erfindungsgemäße Peptid kann nach bekannten Verfahren hergestellt werden.

So kann zur Herstellung ein chemisch-synthetisches Verfahren, zum Beispiel Festphasen-Synthesen oder eine Synthese in flüssigem Medium, herangezogen werden. Bei der Festphasensynthese werden die Aminosäuren in der Abfolge der Sequenz entsprechend den Sequenzprotokollen miteinander verbunden. Die Festphasenpeptidsynthese besteht aus drei wesentlichen Schritten.

1) Aufbau der Aminosäurekette zum Peptid,

2) Spaltung des synthetisierten Peptids vom Harz und

3) Reinigung und Charakterisierung. Für die Synthese der Aminosäurekette sind unterschiedliche Kopplungsmethoden bekannt, wie Sie beispielsweise in "Beyer Walter" 22. Auflage ISBN 3-7776-0485-2 Seite 829-834 beschrieben werden.

Für die Herstellung der erfindungsgemäßen Peptide kann jede Standard-Peptidsynthese eingesetzt werden.

Das Peptid können auch durch Expression der für sie kodierenden Nucleotidsequenzen, zum Beispiel in Chromosomen, Plasmiden

oder anderen Informationsträgern, nackte DNA oder RNA in Organismen, in Zellen oder zellfreien Systemen, hergestellt werden.

Die funktionell für dieses Peptid kodierenden DNA Sequenzen sollen daher von der Erfindung mit umfasst sein.

Gegenstand der Erfindung sind daher auch die Nucleinsäuren, welche für das Peptid gemäß der Aminosäuresequenz gemäß Sequenzprotokoll 1 kodiert, einschließlich aller Allelvariationen.

Das Peptid gemäß Seq. Nr.1 kann durch die DNA-Sequenz gemäß Seq. Nr.2 hergestellt werden, indem die DNA exprimiert wird.

Der erfindungsgemäße Wirkstoff und die pharmazeutische Zusammensetzung können als flüssige, halbfeste oder feste Arzneiformen und in Form von z. B. Injektionslösungen, Tropfen, Säften, Sirupe, Spray, Suspensionen, Granulaten, Tabletten, Pellets, transdermalen therapeutischen Systemen, Kapseln, Pflastern, Zäpfchen, Salben, Cremes, Lotionen, Gelen, Emulsionen oder Aerosolen vorliegen und verabreicht werden, und enthalten die erfindungsgemäßen Peptide in einer physiologisch verträglichen Form und in Abhängigkeit des Applikationsweges pharmazeutischer Hilfsstoffe, wie z. B. Trägermaterialien, Füllstoffe, Lösungsmittel, Verdünnungsmittel, oberflächenaktive Stoffe, Farbstoffe, Konservierungsstoffe, Sprengmittel, Gleitmittel, Schmiermittel, Aromen und/oder Bindemittel. Diese Hilfsmittel können beispielsweise sein: Wasser, Ethanol, 2-Propanol, Glycerin, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokosnussöl, Erdnussöl, Sojabohnenöl, Lecitin, Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumoxid, Titanoxid, Titandioxid, Magnesiumsulfat, Zinksulfat, Calciumsulfat, Pottasche, Calciumphosphat, Dicalciumphosphat, Kaliumbromid, Kaliumjodid, Talkum, Kaolin, Pectin, Crospovidon, Agar und Bentonit.

Die Auswahl der Hilfsstoffe sowie der einzusetzenden Mengen derselben hängt davon ab, ob das Medikament oral, subkutan, parenteral, intravenös, pulmonal, intraperitoneal, transdermal, intramuskulär, nasal, buccal, rectal oder auf andere geeignete Weise appliziert werden soll. Für die orale Applikation eignen sich u. a. Zubereitungen in Form von Tabletten, Dragees, Kapseln, Granulaten, Tropfen, Säften und Sirupe, für die parenterale, topische und inhalative Applikation Lösungen, Suspensionen, leicht rekonstruierbare Pulver zur Inhalation sowie Sprays. In geeigneten perkutanen Applikationsformen kann der Wirkstoff in einem Depot in gelöster Form oder in einem Pflaster, gegebenenfalls unter Zusatz von die Hautpenetration fördernden Mitteln vorliegen. Rektal, transmucosal, parenteral, oral oder percutan anwendbare Zubereitungsformen können die erfindungsgemäßen Peptide verzögert freisetzen.

In flüssiger Form können die erfindungsgemäßen Peptide beispielsweise intravenös, oral, als Nasenspray, subcutan, intramuskulär, inhalativ oder in oder neben das Rückenmark appliziert werden. Weiterhin können die erfindungsgemäßen Wirkstoffe mittels Salben oder Cremes aufgebracht werden.

Die erfindungsgemäßen Peptide können am Wirkort oder an anderen Orten des Organismus entstehen, nachdem Nucleinsäuren (DNA und RNA oder eine Kombination davon), die für die erfindungsgemäßen Peptide kodieren, in den Organismus eingebracht werden. Dies kann durch virale Vektoren, nackte Nucleinsäure (DNA,RNA), Plasmide, künstliche Viruspartikel, Liposomen erreicht werden, die intravenös, intranasal, oral, rectal, subcutan, intramuskulär, in oder an das Rückenmark in den Körper eingebracht werden.

Beispiele:

Protokoll entsprechend Sequenz Nr.1:

Verwendet wurde eine kommerzielle Phagenbibliothek, welche die Präsentation von 12 randomisierten Aminosäuren erlaubt (Ph.D.-12™ Phage Display Library Kit, New England Biolabs, Frankfurt). Hier ist an das Phagenhüllprotein kodierende gp3-Gen nach der Signalsequenz N-terminal die Bibliothek inseriert. Diese besteht aus $1,9 \times 10^9$ unabhängigen Klonen und ist so amplifiziert und konzentriert, dass in 10 $\mu$l durchschnittlich jede Sequenz in 10 Kopien vorliegt. Zur Durchführung der Selektion gegen die SH3 Domäne der menschlichen T-Zell Tyrosinkinase (Lck SH3) wurde rekombinant hergestellte Lck SH3 als Fusion mit Glutathion-S-Transferase (GST-Lck-SH3) in einer Konzentration von 100 $\mu$g/ml in Tris-Bis-Puffer (20 mM Tris-Bis, 150 mM NaCl, pH 6,5) in einer Mikrotiterplattenvertiefung immobilisiert. Hierfür wurden jeweils 200 $\mu$l Proteinlösung über Nacht bei 4°C unter sanftem Schütteln inkubiert. Nach Entfernen der Lösung wurden verbleibende Bindungsstellen der Mikrotiterplattenvertiefung mit BSA (10 mg/ml in Tris-Bis) blockiert (60 Minuten, Inkubation unter leichtem Schütteln). Danach wurden 6 Waschschritte mit 200 $\mu$l Tris-Bis durchgeführt. Für die Selektion wurden 10 $\mu$l Phagen der kommerziellen Phagenbibliothek in 100 $\mu$l Tris-Bis mit 0,1 % Tween (Tris-BisT) für 20 Minuten unter leichtem Schütteln in einer GST-Lck-SH3-beschichteten Mikrotiterplattenvertiefung inkubiert. Die nicht gebundenen Phagen wurden verworfen und anschließend 10 mal mit 200 $\mu$l Tris-BisT gewaschen. Die Elution erfolgte mit 100 $\mu$l 0,2 M Glycin-HCl, pH 2,2 für 10 Minuten unter leichtem Schütteln. Das Eluat wurde durch Zugabe von 25 $\mu$l 1 M Tris-HCl, pH 9,1 neutralisiert. Zur Amplifikation der im Eluat enthaltenen Phagen wurde 9/10 des Eluats zu 20 ml E. coli-Kultur ($OD_{600}$ von 0,1) gegeben und für 5 Stunden bei 37 °C unter starker Belüftung inkubiert. Anschließend wurde die Kultur 20 Minuten bei 5000 rpm zentrifugiert. Der Überstand wurde überführt und 1/6 Volumen PEG/NaCl (20% Polyethylenglycol-8000, 2,5 M NaCl) zugegeben. Der Ansatz wurde nun über Nacht auf Eis gefällt. Danach wurde 60 Minuten bei 5000 rpm zentrifugiert, der Überstand verworfen und das Pellet in 1 ml TBS (50 mM Tris-HCl, pH 7, 5, 150 mM NaC1) resuspendiert. Anschließend wurde 5 Minuten bei 10000 rpm zentrifugiert. Der Überstand wurde überführt und 1/6 Volumen PEG/NaCl zugegeben. Der Ansatz wurde 1 Stunde auf Eis gefällt. Abschließend wurde 60 Minuten zentrifugiert und das Pellet in 100 $\mu$l TBS resuspendiert. Die so erhaltenen Phagen konnten nun in der nächsten Runde eingesetzt werden. Es wurden drei Selektionsrunden durchgeführt. Anschließend wurden willkürlich ausgewählte Phagenklone isoliert und die Aminosäuresequenz der auf diesen Phagen präsentierten Peptide über den Umweg der DNA-Sequenzierung des im Phagengenom kodierten Peptids bestimmt.

Peptid PD 1 (HSKYPLPPLPSL) Sequenz Nr. 1 wurde am Ende der Selektion von 11 der insgesamt 18 charakterisierten Phagenklonen präsentiert, die zugehörige DNA-Sequenz ist Sequenz Nr.2

Charakterisierung der Lck-SH3 Bindungseigenschaften von PD1 Seq.1)

A. "anti-Phage-ELISA" - Bestimmung der relativen Lck-SH3-Bindungsaktivität einzelner Phagenklone mit PD1 (Seq. Nr.1)

Dieser Bindungstest ist ein häufig verwendeter Test zur schnellen Bestimmung relativer Bindungsstärken selektierter Peptide, die hierbei noch physikalisch mit dem Phagen verknüpft vorliegen. Dieser Schritt dient auch zur Eliminierung von (bei dieser Methode nicht selten auftretenden) Phagenvarianten, die z. B. aufgrund ihrer Affinität zur Plastikoberfläche der verwendeten Mikrotiterplatten, angereichert werden und die somit zur Verfälschung der Ergebnisse führen können. Eine grundsätzliche Beschreibung der Methode findet sich z. B. unter Sparks et al. Methods Enzymol. 1995; 255:498-509.

Protokoll:

GST-Lck-SH3 Protein wurde in einer Konzentration von 10 $\mu$g/ml in Tris-Bis über Nacht bei 4°C in Mikrotiterplatten immobilisiert. Verbleibende Bindungsstellen wurden durch 2-stündige Inkubation mit Blockierungspuffer (Bis-Tris mit 2 % Magermilchpulver) abgesättigt. In einer anderen Mikrotiterplatte wurden zuerst je 100 $\mu$l Suspension der zu testenden Phagenvariante mit 100 $\mu$l Blockierungspuffer für 20 Minuten vorinkubiert und erst dann für 1 Stunde zum immobilisierten GST-Lck-SH3 Protein gegeben. Nach einem Waschschritt mit Bis-Tris wurde ein Meerrettich-Peroxidase-konjugierter Antikörper zugegeben und die Platten für eine weitere Stunde inkubiert. Nach weiteren drei Waschschritten mit Bis-Tris konnten die gebundenen Phagen mittels einer Farbreaktion unter Verwendung von 3,3',5,5'-Tetramethylbenzidine/Wasserstoffperoxid nach Abstoppen mit 100 $\mu$l Schwefelsäure bei 450 nm detektiert werden (Brüsselbach S, Korn T, Völkel T, Müller R, Konterman RE. Enzyme recruitment and tumor cell killing in vitro by a secreted bispecific single-chain diabody. Tumor Targeting 1999; 4:115-123). Die Intensität der Gelbfärbung wurde mittels eines Fluostar Optima Mikrotiterplatten-Lesegerätes (BMG Labtechnologies GmbH, Offenburg) dokumentiert und gibt die relative Bindungsstärke der getesteten Phagenvariante an.

B. Fluoreszenztitration - Bestimmung von Bindungskonstanten mit PD1. (Seq. Nr. 1)

Die Bestimmung der Lck-SH3-Bindungsstärke sowie der Bindungsstärke an die SH3-Domänen aus Hck, Abl, PI3K des Peptides PD1 durch Messung der Änderung der intrinsischen Tryptophanfluoreszenz basiert auf der Wechselwirkung von PD1 mit bestimmten konservierten aromatischen Aminosäureresten (hauptsächlich Tryptophan) in der Peptidbindungstasche der SH3-Domäne. Die Messungen wurden im Wesentlichen wie bei Schweimer K, Hoffmann S, Bauer F, Friedrich U, Kardinal C, Feller SM, Biesinger B, Sticht H. Structural investigation of the binding of a herpesviral protein to the SH3 domain of tyrosine kinase Lck. Biochemistry. 2002 Apr 23;41(16):5120-30 beschrieben unter Verwendung eines Perkin-Elmer LS 55 Fluoreszenzspektrometers bei einer Anregungswellenlänge von 290 nm und einer Emissionswellenlänge von 345 nm durchgeführt. Die Titrationskurven, die zur Bestimmung der Bindungskonstanten benötigt werden, wurden durch graduelle Zugabe von PD 1 (aus einer Peptidstammlösung in Wasser) zu 1,6 ml mit 0,5 µM der freien Lck-SH3 Domäne (ohne GST-Fusion) bzw. der anderen SH3-Domänen in PBS, 1 mM DTT gegeben. Nach PD1-Zugabe wurde jeweils die resultierende Fluoreszenzänderung aufgezeichnet. Durch eine Kontrolltitration (Hintergrundmessung), bei welcher die Peptidlösung gegen Wasser ersetzt wurde, konnten die Abweichungen der Fluoreszenzwerte, welche durch eine sinkende SH3-Endkonzentration während der Titration hervorgerufen wurden, ausgeglichen werden. Da PD1 einen Tyrosinrest in der Peptidsequenz enthält, wurden Hintergrundmessungen durchgeführt, bei welchen pro Titrationsschritt Tyrosinamid in der gleichen Konzentration wie PD1 eingesetzt wurde. Die erhaltenen experimentellen Daten wurden unter Verwendung von Gleichung 1 angepasst, wobei $L_{ges}$ der absoluten Peptid- (Liganden) und $P_{ges}$ der absoluten SH3-(Protein) Konzentration pro Messpunkt entspricht, F ist die gemessene Fluoreszenzintensität für jede PD1-Konzentration und $F_{max}$ ist die maximale beobachtete Fluoreszenzintensität des Proteins bei Sättigung mit PD1, $F_{min}$ ist die Fluoreszenzintensität des Proteins bei Titrationsstart.

Gleichung 1:

$$F = F_{min} + \left( \frac{[P_{ges}] + [L_{ges}] + K_d}{2} - \sqrt{\frac{([P_{ges}] + [L_{ges}] + K_d)^2}{4} - [P_{ges}] * [L_{ges}]} \right) * \frac{(F_{max} - F_{min})}{P_{ges}}$$

Durch nichtlineare Regression mit $F_{max}$ und $K_d$ als angepasste Parameter konnten die experimentellen Daten an obige Gleichung angepasst werden.

C. NMR Spektroskopie - Nachweis der HIV1-Nef-Verdrängung von Lck- und Hck-SH3 durch Peptidliganden mit PD1.

Kernmagnetische (NMR) Resonanzspektren wurden bei einer Protonenfrequenz von 600 MHz bei 298 K mit einem Varian Unity INOVA Spektrometer aufgenommen. Die Bindung von Liganden (PD1, HIV1-Nef) an die Hck-SH3 Domäne wurde anhand bei Komplexbildung auftretenden, von der Ligandenkonzentration abhängigen, chemischen Verschiebungsänderungen verfolgt. Die dafür notwendigen Daten wurden durch eine Titrationsreihe heteronuklearer Einquantenkohärenz-Experimente ($^1$H, $^{15}$N HSQC-Experimente) unter Verwendung von $^{15}$N-markiertem Hck-SH3-Protein mit jeweils steigender Liganden-Konzentration ermittelt. Die Anfangskonzentration an Hck-SH3 lag dabei zwischen 200 und 400 µM (PBS, 1mM β-Mercaptoethanol). PD1-Stammlösungen lagen in Konzentrationen zwischen 5 und 10 mM vor. Titrationen wurden bis zu einem 4-fachen Überschuss an Peptid durchgeführt. Die HIV1 Nef-Stammlösung lag in einer Konzentration von 1,6 mM (PBS, 14 mM β-Mercaptoethanol) vor, die entsprechenden Titrationen erfolgten bis zu einem Verhältnis von 1:1. Um eine HIV1-Nef-Verdrän gung von der Hck-SH3 Domäne nachzuweisen, wurden alle dazu nötigen $^1$H, $^{15}$N HSQC-Experimente mit Ligandenkonzentrationen im Sättigungsbereich durchgeführt. Im Einzelnen wurde A. die freie Hck-SH3-Domäne vorgelegt und zu dieser HIV 1-Nef titriert, B. ein Hck-SH3-HIV 1-Nef-Komplex vorgelegt und zu diesem Peptid PD1 titriert und C. die freie Hck-SH3-Domäne vorgelegt und zu dieser Peptid PD1 titriert.
(Novus Biologicals, Inc., Littleton, USA) in 20 ml Blocking Puffer für je 2 h erfolgte dreimaliges Waschen mit T-TBS für je 20 min. Alle bisherigen Schritte wurden unter Schütteln durchgeführt. Die Detektion erfolgte am Gel Doc 2000 Gerät (Bio-Rad, München) 1 min nach Zugabe des Substrates Chemiluminescence Western Blotting Reagent (Pierce Biotechnology, Inc., Rockford, USA). Die Spot-Intensitäten wurden mit der Quantity-One Software (Bio-Rad, München) ausgewertet. Die Analyse der Spot-Intensitäten erfolgte nach Subtraktion des Hintergrundes.

E. Ergebnisse:

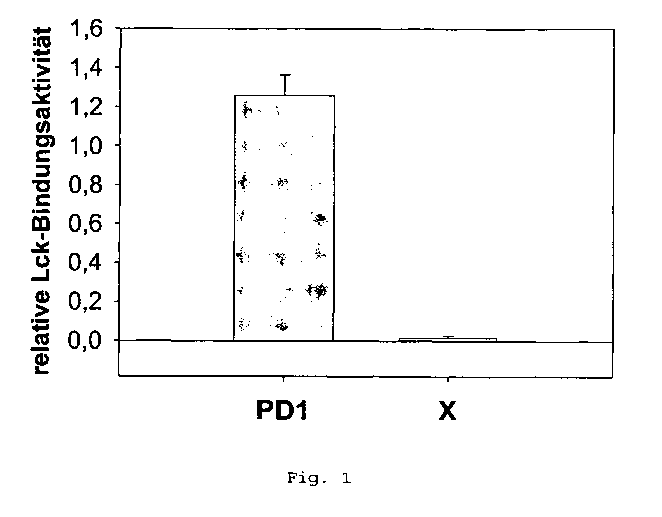

Fig. 1 zeigt Bestimmung der relativen Lck-SH3-Bindungsaktivität des Peptids PD1 (Seq.1) mittels "anti-Phage"-ELISA. X ist hier das nicht bindende Peptid HTLQIPQHATSF Es wurde die relative Bindungsaktivität von PD1-präsentierenden Phagenvarianten an die Lck-SH3 bestimmt. Gezeigt sind Mittelwert und Standardabweichung aus insgesamt 22 Einzelexperimenten. Zum Vergleich ist die Lck-Bindungsaktivität eines nicht bindenden Peptides HTLQIPQHATSF aufgetragen. Hierzu wurden 7 Einzelexperimente durchgeführt. Unter den Balken sind die Namen der Peptide angegeben, die von den jeweiligen, selektierten Phagen präsentiert werden.

Tabelle 1: Bestimmung der SH3-PD1-Bindungskonstanten (seq.1).

Bestimmung der Bindungskonstanten ($K_D$) für den Komplex aus Peptid PD1 und Lck-SH3 sowie weiterer SH3-Domänen durch Fluoreszenztitration. Jedes Experiment wurde zweimal durchgeführt.

| SH3 Domäne | $K_d$ ($\mu$M) |
|---|---|
| Lck | 48.66 $\pm$ 1.65 |
| Hck | 0.23 $\pm$ 0.03 |
| PI3K | 112.14 $\pm$ 10.08 |
| Abl | >> 300 |

Abkürzungen: Hck: hematopoethische Zell-spezifische Kinase, PI3K: Phosphoinositol-3-Kinase, Abl: Abelson-Tyrosinkinase

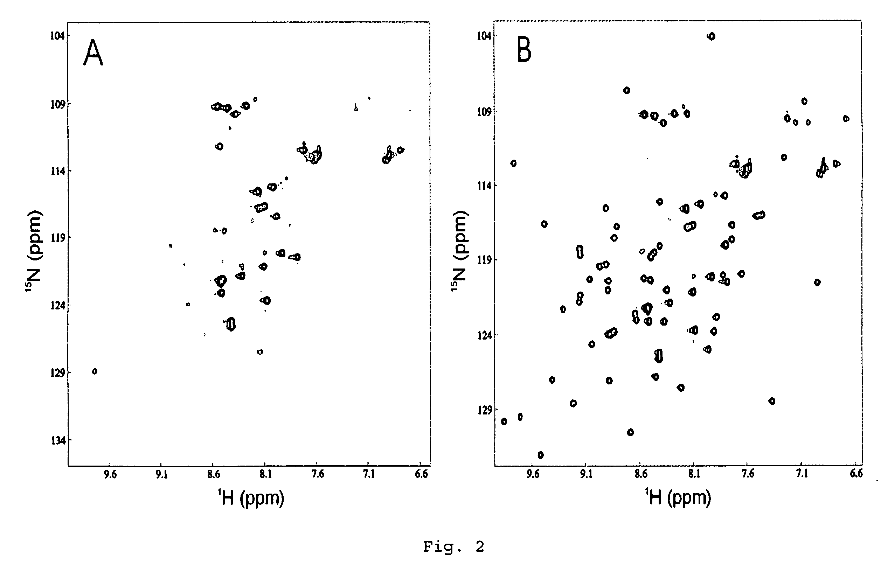

Fig.2 zeigt den Nachweis der HIV1-Nef-Verdrängung von Hck-SH3 durch PD1 mittels NMR-Spektroskopie. Alle nötigen $^{1}$H, $^{15}$N HSQC-Experimente wurden mit Ligandenkonzentrationen im Sättigungsbereich durchgeführt. Zu erkennen sind jeweils die Signale der $^{15}$N-markierten Hck-SH3-Domäne. (A) Die freie Hck-SH3-Domäne wurde vorgelegt und zu dieser HIV1-Nef im Verhältnis 1:1 titriert. Dargestellt ist der Endpunkt der Titration. (B) Zu dem Hck-SH3-HIV 1-Nef-Komplex aus (A) wurde das Peptid PD1 zutitriert. Die daraus entstandenen Signale entsprechen im Wesentlichen denen eines Hck-SH3-PD1-Komplexspektrums.

Sequenzprotokolle:

PD1 entspricht Sequenz_1

Sequenz Nr. 2 entspricht DNA-Sequenz von Sequenz Nr.1

Nutzen der Erfindung:

Im Speziellen kann die Pathogenität solcher viraler Erreger geschwächt werden, die direkt Effektormoleküle, welche an die SH3-Domäne von Lck (Hck) binden, besitzen. Hierzu gehören z. B. die menschlichen Krankheitserreger HIV-1 (Nef-Protein) und Hepatitis C (NS5A Protein).

Krankheiten: HIV1, Hepatitis C und Autoimmunkrankheiten

Überraschenderweise wurde für PD1 eine hochaffine Bindung an die SH3-Domäne der hematopoethischen Zell-spezifischen Kinase (Hck) festgestellt (siehe Tab.1, $K_D$ ca. 0,2 $\mu$M). Die Selektivität der PD1-Bindung an Lck und Hck konnte durch dessen schlechte Bindungseigenschaften an die nicht zum Src-Typ gehörenden Kinasen PI3K und Abl nachgewiesen werden (Tab1, $K_D$s von ca. 129 und >>300 $\mu$M).

Lck und Hck sind beides Modulatoren der Immunantwort, wirken jedoch in unterschiedlichen Zelltypen. Lck liegt ausschließlich in T- und Killer-Zellen vor, Hck wird in Monocyten/Makrophagen (Ziegler et al. Novel protein-tyrosine kinase gene (hck) preferentially expressed in cells of hematopoietic origin Mol Cell Biol. 1987 Jun;7(6):2276-85) aber auch in Neutrophilen, Eosinophilen, dentritischen Zellen und Osteoklasten exprimiert.

Die menschliche hematopoetische-Zell Tyrosinkinase (Hck) ist in einer Vielzahl von Signalwegen involviert. Ihr wird u. a. eine wichtige Rolle während der Phagocytose (Suzuki et al. Differential involvement of Src family kinases in Fc gamma receptor-mediated phagocytosis. J. Immunol. 2000 Jul 1; 165(1):473-82), bei der FcγRII-Rezeptor (Ghazizadeh et al. Physical and functional association of Src-related protein tyrosine kinases with Fc gamma RII in monocytic THP-1 cells, J. Biol. Chem. 1994 Mar 25; 269(12):8878-84) bzw. Integrinvermittelten Signalübertragung (Mocsai et al. Adhesiondependent degranulation of neutrophils requires the Src family kinases Fgr and Hck. J. Immunol. 1999 Jan 15; 162(2):1120-6) und der Chemotaxis von Monocyten (Kumar et al. Soluble E-selectin induces monocyte chemotaxis through Src family tyrosine kinases, J. Biol. Chem. 2001 Jun 15; 276(24):21039-45) zugeschrieben.

Peptidliganden der Hck-SH3-Domäne können somit ebenfalls zur Unterdrückung der Immunantwort eingesetzt werden, wie es bei Organtransplantationen notwendig ist.

Wie auch bei der Lck kann die Komplexbildung von exogenen oder transformierten zelleigenen Liganden mit der SH3-Domäne der Hck zu deren Deregulierung und somit zu schweren Störungen von Abläufen innerhalb der normalen Immunabwehr der betroffenen Zelltypen kommen. Die Interaktion des Nef-Proteins aus HIV-1 mit der SH3-Domäne der Hck gehört dabei zu einem der stabilsten SH3-Ligandenkomplexe, die bislang beschrieben wurden (Lee et al. A single amino acid in the SH3 domain of Hck determines its high affinity and specificity in binding to HIV-1 Nef protein. EMBO J. 1995 Oct 16;14(20):5006-15). Es gibt Hinweise, dass die Interaktion von Nef mit Hck-SH3 an der Ausprägung des durch HIV-1 verursachten Krankheitsbildes AIDS beteiligt ist (Hanna et al. The pathogenicity of human immunodeficiency virus (HIV) type 1 Nef in CD4C/HIV transgenic mice is abolished by mutation of its SH3-binding domain, and disease development is delayed in the absence of Hck. J Virol. 2001 Oct;75(19):9378-92).

Die ermittelte Bindungsstärke des PD1-Hck-SH3 (Tab. 1) liegt in der gleichen Größenordnung, wie er für einen Komplex aus Hck-SH3 mit dem HIV-1 Nef Protein bekannt ist. Durch NMR-Experimente (Abb. 2) wurde nachgewiesen, dass - zumindest in vitro - eine effektive Verdrängung des viralen Nef-Proteins von der Hck-SH3 Domäne und somit eine Therapie von AIDS möglich ist.

**Patentansprüche**

1. Peptid gemäß der Sequenz Nr.1.

**2.** Arzneimittel,
**dadurch gekennzeichnet,**
**dass** es das Peptid gemäß Anspruch 1 enthält.

**3.** Arzneimittel nach Anspruch 2,
**dadurch gekennzeichnet, dass** es ein Mittel zur Linderung und/oder Heilung der Krankheiten AIDS, Hepatitis, Multiple Sklerose, systemischer Lupus erythematodes, Diabetes, rheumatische Arthritis, chronische Thyreoiditis, Spondylitis ankylosans, anteriore Uveitis, Goodparteure Syndrom, Basedow Krankheit, Myasthenia gravis, Krebs und Pemphigus vulgaris ist.

**4.** Lck-SH3 Komplex
**dadurch gekennzeichnet,**
**dass** sein Komplexpartner das Peptid nach Anspruch 1 ist.

**5.** Lck-SH3 Komplex nach Anspruch 4,
**dadurch gekennzeichnet**
das er ein Komplex einer SH3-Domäne vom Menschen, von der Maus, der Ratte, dem Hund, dem Nachtaffen, dem Schimpansen oder einer Spezies ist, welche eine SH3-Domäne besitzt, welche mit der des Menschen identisch ist.

**6.** Hck-SH3 Komplex
**dadurch gekennzeichnet,**
**dass** sein Komplexpartner das Peptid nach Anspruch 1 ist.

**7.** Hck-SH3 Komplex nach Anspruch 6,
**dadurch gekennzeichnet**
das er ein Komplex einer SH3-Domäne vom Menschen, vom Schimpansen, dem Langschwanzmakak, dem Hund oder einer Spezies ist, welche eine SH3-Domäne besitzt, welche mit der des Menschen identisch ist.

**8.** Nukleotidsequenz nach Sequenz Nr. 2.

**Claims**

**1.** Peptide according to the sequence no. 1.

**2.** Medicine
**characterised in that** it contains the peptide according to claim 1.

**3.** Medicine according to claim 2,
**characterised in that** this an agent for the alleviation and / or curing of the diseases AIDS, hepatitis, multiple sclerosis, systemic lupus erythematosus, diabetes, rheumatoid arthritis, chronic thyroiditis, ankylosing spondylitis, anterior uveitis, Goodpasture syndrome, Basedow's disease, myasthenia gravis, cancer and pemphigus vulgaris.

**4.** LcK-SH3 complex
**characterised in that**
its complex partner is the peptide according to claim 1.

**5.** LcK-SH3 complex according to claim 4,
**characterised in that**
it is a complex of an SH3 domain of humans, mice, rats, dogs, owl monkeys, chimpanzees or a species, which has an SH3 domain that is identical to that of humans.

**6.** HcK-SH3 complex
**characterised in that**
its complex partner is the peptide according to claim 1.

**7.** HcK-SH3 complex according to claim 6,
**characterised in that**

it is a complex of an SH3 domain of humans, chimpanzees, long-tailed macaques, dogs or a species, which has an SH3 domain that is identical to that of humans.

8. Nucleotide sequence according to sequence no. 2.

**Revendications**

1. Peptide suivant la séquence numéro 1.

2. Médicament,
   **caractérisé,**
   **en ce qu'**il contient le peptide suivant la revendication 1.

3. Médicament suivant la revendication 2,
   **caractérisé, en ce qu'**il est un moyen pour soulager et/ou guérir les maladies sida, hépatite, sclérose en plaques, lupus érythémateux aigu disséminé, diabète, polyarthrite rhumatoïde thyréoïdite chronique, spondylarthrite ankylosante, uvéite antérieure, syndrome de Goodparteure, maladie de Basedow, myasthénie grave, cancer et pemphigus vulgaire.

4. Complexe Lck-SH3
   **caractérisé**
   **en ce que** son partenaire de complexe et le peptide suivant la revendication 1.

5. Complexe Lck-SH3 suivant la revendication 4,
   **caractérisé,**
   **en ce que** c'est un complexe d'un domaine SH3 de l'homme, de la souris, du rat, du chien, du singe de nuit, du chimpanzé ou d'une espèce qui a un domaine SH3 qui est identique à celui de l'homme.

6. Complexe Lck-SH3
   **caractérisé,**
   **en ce que** son partenaire de complexe est le peptide suivant la revendication 1.

7. Complexe Lck-SH3 suivant la revendication 6,
   **caractérisé**
   **en ce que** c'est un complexe d'un domaine SH3 de l'homme, du chimpanzé, du macaque à longue queue, du chien ou d'une espèce qui a un domaine SH3 qui est identique à celui de l'homme.

8. Séquence de nucléotide suivant la séquence numéro 2.

Fig. 1

Fig. 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **POGHOSYAN Z. et al.** Phosphorylationdependent interactions between ADAM15 cytoplasmic domain and Scr family protein-tyrosine kinase. *J Biol Chem.,* 15. Februar 2002, vol. 277 (7), 4999-5007
- Beyer Walter. 829-834
- **SPARKS et al.** *Methods Enzymol.,* 1995, vol. 255, 498-509
- **BRÜSSELBACH S ; KORN T ; VÖLKEL T ; MÜLLER R ; KONTERMAN RE.** Enzyme recruitment and tumor cell killing in vitro by a secreted bispecific single-chain diabody. *Tumor Targeting,* 1999, vol. 4, 115-123
- **SCHWEIMER K ; HOFFMANN S ; BAUER F ; FRIEDRICH U ; KARDINAL C ; FELLER SM ; BIESINGER B ; STICHT H.** Structural investigation of the binding of a herpesviral protein to the SH3 domain of tyrosine kinase Lck. *Biochemistry,* 23. April 2002, vol. 41 (16), 5120-30
- **ZIEGLER et al.** Novel protein-tyrosine kinase gene (hck) preferentially expressed in cells of hematopoietic origin. *Mol Cell Biol.,* Juni 1987, vol. 7 (6), 2276-85
- **SUZUKI et al.** Differential involvement of Src family kinases in Fc gamma receptor-mediated phagocytosis. *J. Immunol.,* 01. Juli 2000, vol. 165 (1), 473-82

- **GHAZIZADEH et al.** Physical and functional association of Src-related protein tyrosine kinases with Fc gamma RII in monocytic THP-1 cells. *J. Biol. Chem.,* 25. Marz 1994, vol. 269 (12), 8878-84
- **MOCSAI et al.** Adhesiondependent degranulation of neutrophils requires the Src family kinases Fgr and Hck. *J. Immunol.,* 15. Januar 1999, vol. 162 (2), 1120-6
- **KUMAR et al.** Soluble E-selectin induces monocyte chemotaxis through Src family tyrosine kinases. *J. Biol. Chem.,* 15. Juni 2001, vol. 276 (24), 21039-45
- **LEE et al.** A single amino acid in the SH3 domain of Hck determines its high affinity and specificity in binding to HIV-1 Nef protein. *EMBO J.,* 16. Oktober 1995, vol. 14 (20), 5006-15
- **HANNA et al.** The pathogenicity of human immunodeficiency virus (HIV) type 1 Nef in CD4C/HIV transgenic mice is abolished by mutation of its SH3-binding domain, and disease development is delayed in the absence of Hck. *J Virol.,* Oktober 2001, vol. 75 (19), 9378-92

SEQUENCE LISTING

<110> Forschungszentrum Jülich GmbH

<120> An die Lck-HS3 und Hck-SH3 bindende Peptide

<130> PT1.2218EP

<160> 2

<170> PatentIn version 3.1

<210> 1
<211> 12
<212> PRT
<213> Artificial Sequence

<220>
<223> Die Sequenz wurde aus einer Phagenbibliothek erhalten

<400> 1

```
His Ser Lys Tyr Pro Leu Pro Pro Leu Pro Ser Leu
1               5               10
```

<210> 2
<211> 36
<212> DNA
<213> Artificial sequence

<220>
<223> Die Sequenz entspricht der der Sequenz Nr.1 zugeordneten DNA Sequ enz

<400> 2
cattctaagt atccgctgcc tccgttgccg tctctt          36

SEQUENCE LISTING

<110> Forschungszentrum Jülich GmbH

<120> An die Lck-HS3 und Hck-SH3 bindende Peptide

<130> PT1.2218EP

<160> 2

<170> PatentIn version 3.1

<210> 1
<211> 12
<212> PRT
<213> Artificial sequence

<220>
<223> Die Sequenzwurde aus einer Phagenbibliothek erhalten

<400> 1

```
          His Ser Lys Tyr Pro Leu Pro Pro Leu Pro Ser Leu
          1               5                   10
```

<210> 2
<211> 36
<212> DNA
<213> Artificial sequence

<220>
<223> Die Sequenz entspricht der der Sequenz Nr.1 zugeordneten DNA Sequ enz

<400> 2
cattctaagt atccgctgcc tccgttgccg tctctt          36

SEQUENCE LISTING

<110> Forschungszentrum Jülich GmbH

<120> An die Lck-SH3 und Hck-SH3 Domäne bindende Peptide

<130> PT1.2218EP

<160> 4

<170> Patent In version 3.3

<210> 1
<211> 12
<212> PRT
<213> Artificial sequence

<220>
<223> Oligopeptide

<400> 1

```
           His Ser Lys Tyr Pro Leu Pro Pro Leu Pro Ser Leu
           1               5                   10
```

<210> 2
<211> 12
<212> PRT
<213> Artificial Sequence

<220>
<223> Oligopeptide

<400> 2

```
           His Ser Lys Arg Pro Leu Pro Pro Leu Pro Ser Leu
           1               5                   10
```

<210> 3
<211> 36
<212> DNA
<213> Artificial Sequence

<220>
<223> Codierende DNA

<400> 3
cattctaagt atccgctgcc tccgttgccg tctctt          36

<210> 4
<211> 12
<212> PRT
<213> Artificial Sequence

<220>
<223> Oligopeptide

<400> 4

```
           His Phe Lys Arg Pro Leu Pro Pro Leu Pro Ser Leu
           1               5                   10
```